# EUROPEAN PATENT SPECIFICATION

(11) **EP 3 430 182 B1**
(45) Date of publication and mention of the grant of the patent: **23.12.2020**
(21) Application number: 17714157.9
(22) Date of filing: 10.03.2017
(51) Int. Cl.: C25B 1/30, C25B 9/08, C25B 9/10, C25B 9/18, C25B 15/08

(54) **ELECTROCHEMICAL CELL FOR GAS-PHASE REACTANT IN LIQUID ENVIRONMENT**
ELEKTROCHEMISCHE ZELLE FÜR GASPHASENREAKTAND IN EINER FLÜSSIGEN UMGEBUNG
CELLULE ÉLECTROCHIMIQUE POUR RÉACTIF EN PHASE GAZEUSE DANS UN ENVIRONNEMENT LIQUIDE

(30) Priority: 17.03.2016 US 201662309655 P
(43) Date of publication of application: 23.01.2019
(73) Proprietor: Hpnow APS, 2400 Copenhagen (DK)
(72) Inventor: VERDAGUER CASADEVALL, Arnau, 2400 København (DK); FRYDENDAL, Rasmus, 2700 Brønshøj (DK)
(74) Representative: Stütz, Jan
(86) International application number: PCT/EP2017/055710
(87) International publication number: WO 2017/157797

(56) References cited:
- JP-A- 2010 001 519
- US-A- 5 645 700
- US-A1- 2014 131 217

## Description

The invention is related to an electrochemical cell with improved properties for production of chemicals from a gas-phase reactant in a liquid environment. The electrochemical cell has a membrane electrode assembly configuration and is comprised of an anode, an ion exchange membrane and a cathode. The membrane electrode assembly is in direct contact with a liquid solution to facilitate extraction and handling of the produced chemicals, and the gas reactant is delivered through the liquid to the membrane electrode assembly.

The present invention also relates to a process using the electrochemical cell for the synthesis of chemicals.

### Background of the invention

Electrochemical methods are a viable strategy to synthesize chemicals on-site, on-demand using compact reactors. For example, they are employed today for the synthesis of chlorine-based compounds (e.g. sodium hypochlorite) used in water treatment and biocide applications, or for the synthesis of CO from CO₂. Typically electrochemical cells are composed of three parts: anode, electrolyte and cathode. An oxidation reaction takes place at the anode, while charged species are transported through the electrolyte and a reduction reaction happens at the cathode.

Of particular interest is the development within the last decades of electrolytes based on ion exchange membranes. These substitute liquid electrolytes, which require extensive handling, can be hazardous and ultimately complicate the separation of generated products. The term electrochemical cell in this document refers to a membrane electrode assembly, whereby the electrolyte is an ion exchange membrane. Standard techniques to build membrane electrode assemblies including catalyst-coated membranes and gas diffusion electrodes are well-known to practitioners in the field (see for example Handbook of Fuel Cells: Fundamentals, Technology and Applications, Wiley VCH, 2014). Membrane electrode assemblies can be comprised of more than one membrane and more than one gas diffusion layer on either side.

The use of ion exchange membranes as electrolytes is widespread for energy applications, for instance in fuel cells and electrolyzers, but thus far has seen limited use for the synthesis of chemicals despite allowing easy separation of reaction products. One of the reasons for such limited use is that chemicals synthesized at the catalyst layer of the electrochemical cell need to be extracted from the membrane electrode assembly. This is particularly challenging when gas-phase reactants need to be fed into the cell, and most solutions proposed in the literature rely purely on diffusion of reactants and products (see for example Handbook of Fuel Cells: Fundamentals, Technology and Applications, Wiley VCH, 2014).

To facilitate the extraction of products some practitioners have turned into immersing the electrodes into liquid solutions. That way, products dissolve into the liquid and diffuse through it. This is widely practiced in liquid electrolytes (see for example U.S. Pat. No. 6,712,949, Gopal), but not utilized in membrane-based electrochemical cells, despite the advantages these electrodes represent in terms of scalability and product separation.

In membrane electrode assembly configurations products formed in the vicinity of the ion exchange membrane need to be extracted through the gas diffusion layer. Particles, for instance from catalyst dispersions, can be present in the gas diffusion layer and decompose products on their way out by chemical or electrochemical means.

One of the key reasons why membrane electrode assemblies are not utilized in a liquid environment is that gas reactants, such as oxygen, hydrogen, CO or CH₄ have very low solubility in most liquids, including aqueous solutions. For example, oxygen solubility in water is only of 40 mg/L under normal conditions. In turn, this restricts availability of reactants at the electrode and achievable current densities, which are typically limited to a few mA per cm2 . This is exemplified in the report by Li et al (Drinking Water Purification by Electrosynthesis of Hydrogen Peroxide in a Power-Producing PEM Fuel Cell, ChemSusChem 2013, Volume6, Issue11, Pages 2137-2143), where current densities reach values up to 30 mA/cm2 .

Yamanaka et al. report an attempt to mitigate the problem addressed at oxygen reduction to hydrogen peroxide (Neutral H202 Synthesis by Electrolysis of Water and 02, Angewandte Chemie 2008, Volume 47, Issue10, Pages 1900-1902) . In this work, half of the electrochemical cell cathode is exposed to gaseous oxygen, and the other half is immersed in liquid electrolyte. Using this method current densities of 20 to 60 mA/cm2 can be achieved. However, faradaic efficiency to hydrogen peroxide is 26 %, which could indicate that hydrogen peroxide removal from the electrode is difficult.

The usual method adopted by practitioners in the art to solve the problems associated with the limited mass transport of gasses in liquids is to operate the electrochemical cells in a fully gaseous environment, with limited liquid interactions.

Examples of this strategy for the production of chemicals are found in WO 2013/045895. In this document a fuel cell aimed at producing hydrogen peroxide is described. Hydrogen peroxide is produced at the cathode of the reactor using pressurized humidified oxygen, without the presence of liquid water (other than water from the membrane or produced at the catalyst) . Current density in this case approaches 80 mA/cm2 , but faradaic efficiency to hydrogen peroxide is not stated.

A similar example is US Pat. No. 7,892,408 B2, disclosing an electrolyzer cell for hydrogen peroxide production using gaseous oxygen or air feeds to the cathode, without the presence of liquid water. Current densities in this case are 200-300 mA/cm2 .

US 5,645,700, JP 2010-1519 and US 2014/0131217 A1 disclose further devices

### Summary of the invention

The present invention was made in view of the prior art described above, and its objective is to facilitate electrochemical production of chemicals in a membrane electrode assembly configuration. Using this invention it is possible to enhance extraction of products from the membrane electrode assembly. The invention also enables an improved utilization of gaseous reactants.

In a first aspect, the present invention provides enhanced mass transport of gas phase reactants to the electrochemical cell and easy extraction of products. This is very useable for the electrochemical synthesis of products from a gaseous reactant in a membrane electrode assembly type electrochemical cell. The gas is delivered to the electrochemical cell by convection through the liquid solution and the gas diffusion layer.

The presence of a liquid solution in immediate contact with the anode and/or cathode of the cell eases extraction of products by facilitating their diffusion to the liquid.

### Detailed description of the invention

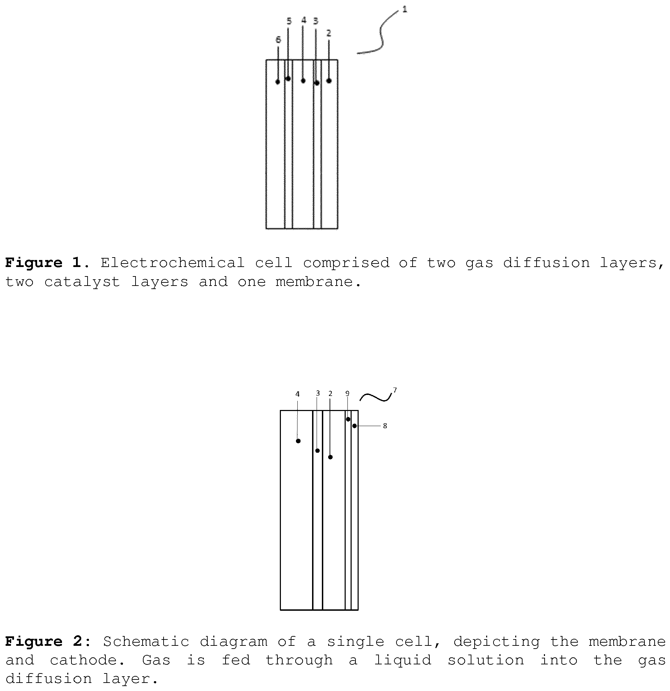

The present invention relates to a novel electrochemical cell design in the membrane electrode assembly configuration and a method for improved transport of reactants. A membrane electrode assembly consists of a cathode and an anode which are pressed against either sides of a membrane, shown in Figure 1. Point number 1 is the membrane electrode assembly. Point number 2 shows the anode gas diffusion layer or current collector, point number 3 the anode catalyst layer. Together these form the anode of the cell. Point number 4 is the ion exchange membrane, preferably a cation exchange perfluorinated membrane. Point number 5 is the cathode catalyst layer, and point number 6 the cathode gas diffusion layer or current collector. Together these form the cathode of the cell. In particular the present invention is directed to a method for improved mass transport of reactants and products in an electrochemical cell. The invention is also related to a method allowing easy extraction of products generated at the electrodes. The invention is further related to optimized use of a gaseous reactant.
Accordingly, the present invention provides a liquid solution in the immediate vicinity of the electrodes to facilitate extraction of the products from the electrochemical cell. The liquid solution can be static or flowing, preferably flowing. The liquid solution is preferably composed of more than 90% of water. The temperature of the liquid solution is between -20 and 500 ºC, preferably between 0 and 100 ºC.
The invention also provides a method to deliver a gaseous reactant through the liquid solution and into the electrode in a manner that gas solubility and diffusion in the liquid is not limiting current density in the cell. The gas is delivered to the electrochemical cell through a gas disperser. An example of a suitable gas disperser could be a tube or other suitable gas conduit, which has a suitable fluidizing media in contact with the electrode to facilitate dispersion of the gas. Using this invention, gas diffusion in the liquid is overcome by convection and mass transport of reactants is enhanced. Suitable gas reactants are well known to those versed in the art, several examples include hydrogen, carbon monoxide, carbon dioxide, methane, propene, oxygen and others. The preferred electrode area covered by the gas disperser is between 10 and 90%, even more preferred between 30 and 70%.

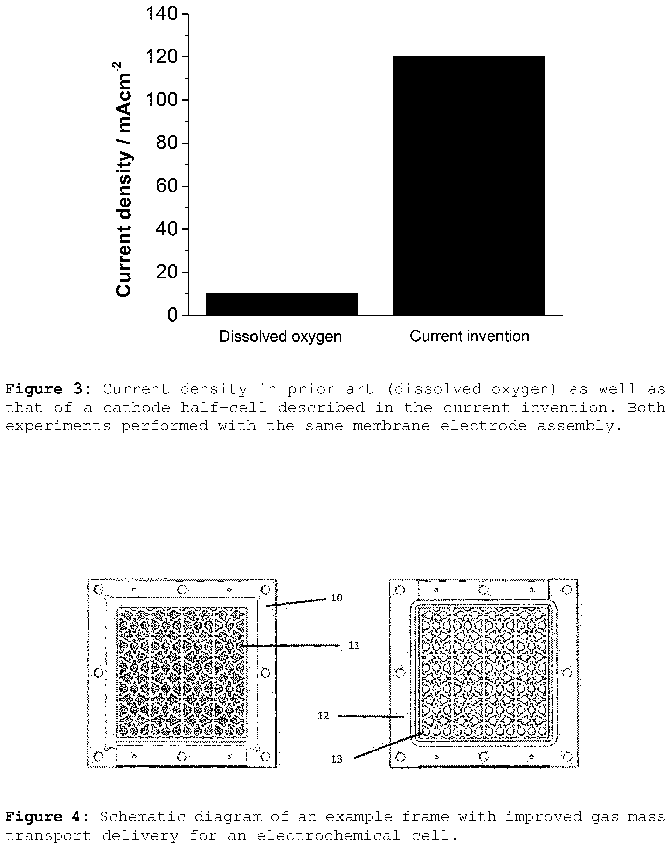

The present invention also relates to a device for the electrochemical production of chemical compounds in an electrochemical cell consisting of a membrane electrode assembly. The membrane electrode assembly is at least partly immersed in a liquid solution, at least on the side of the membrane requiring a gas reactant. For clarity, the membrane electrode assembly may be partly or fully immersed in the liquid solution. Figure 2 shows a diagram of the gas delivery system onto half a membrane electrode assembly, point number 7. Point number 8 shows the gas disperser, preferably delivered through a porous material which allows gas to cross into the liquid layer, depicted in number 9. The gas bubbles formed go on to the gas diffusion layer, 5, and into the catalyst layer, 6. 4 represents the ion exchange membrane separating anode and cathode. An advantage of the present invention over prior art is the enhanced mass transport of gaseous reactants through a liquid solution, which enables substantially higher current densities at the electrode, as shown in Figure 3, while the products can be easily carried away by the liquid. The electrodes of the membrane electrode assembly can be catalyst-coated membranes or gas diffusion electrodes or a mixture of the two. As an example, one could have a membrane electrode assembly with a gas diffusion electrode on the cathode side and a catalyst-coated membrane on the anode side.

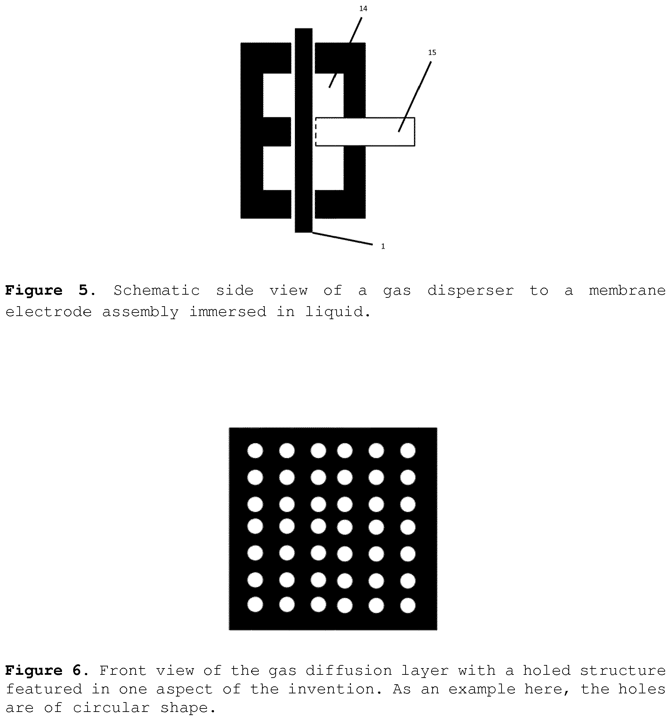

Figure 4 shows a frame containing gas dispersers that can be coupled to an electrochemical cell. The frame, marked by point 10, is to be pressed against one side of a membrane electrode assembly, and it has a volume available for a liquid and gas delivery channels. The structures marked by point 11 host the gas dispersers and may contain a piece of porous material or other suitable fluidizing media to enhance dispersion of the gas. With a slight gas overpressure, the gas is driven through the porous material and directed into the gas diffusion layer in a highly efficient manner. The frame indicated by point 12 is used on the other side of the electrochemical cell. This frame contains structures, point 13, for counter pressure and may or may not include the gas delivery system described above. Figure 5 shows a side view diagram of a gas delivery system. Point 14 indicates a volume for liquid, point 1 the membrane electrode assembly and point 15 a gas disperser. The gas disperser could be a tube or it could also contain one or more porous layers to act as fluidizing media. These are helpful to facilitate dispersion of the gas into the liquid. In this configuration the MEA can be partly or fully immersed in liquid for optimal extraction of generated products while the gas channel in close vicinity to the MEA provides improved gas delivery.

Another aspect of the invention, which could be combined with the method allowing for improved gas delivery, is the utilization of a gas diffusion layer facilitating extraction of the products generated at the electrode. At least one of the gas diffusion layers in the membrane electrode assembly is made of carbon, titanium or other suitable materials, and presents openings (for example through holes) in a pattern, which can be regular, semi-regular or random. The shape of the holes is not of importance to the present invention, and their dimensions are preferably between 1 pm and 5000 pm. Figure 6 shows a schematic representation of the holes in the gas diffusion layer, depicted as a manner of example in a circular shape. A gas diffusion layer with this hole distribution facilitates extraction of the products generated at the electrode by providing an obstacle-free path to the molecules. This gas diffusion layer can be combined with a gas diffusion electrode, a catalyst-coated membrane or both. The gas diffusion layer, either as received or modified in certain ways, can be further treated to modify its interaction with water. For example, hydrophobicity can be promoted by adding polytetrafluoroethylene (PTFE) or other fluorocarbon polymers (e.g. polyvinylidene fluoride or polyvinylfluoride) in a way well-known to practitioners in the art. Hydrophilicity can be promoted in a similar manner by adding hydrophilic molecules.
Another strategy to increase removal efficiency of products from the catalyst layer, which could be combined with the previously described inventions, is to selectively deposit catalyst on a gas diffusion layer (forming a gas diffusion electrode) or on a membrane (forming a catalyst-coated membrane) in selected regions to facilitate extraction of the products generated at the electrode. This can also decrease residence time of products at the gas diffusion layer, minimizing their possible decomposition by the electrode. This selective coating can be achieved for instance by using a pattern when depositing the catalyst ink. As an example, the pattern could have between 5 and 80 % of open area and a distance between coated areas between 10 and 10000 pm.

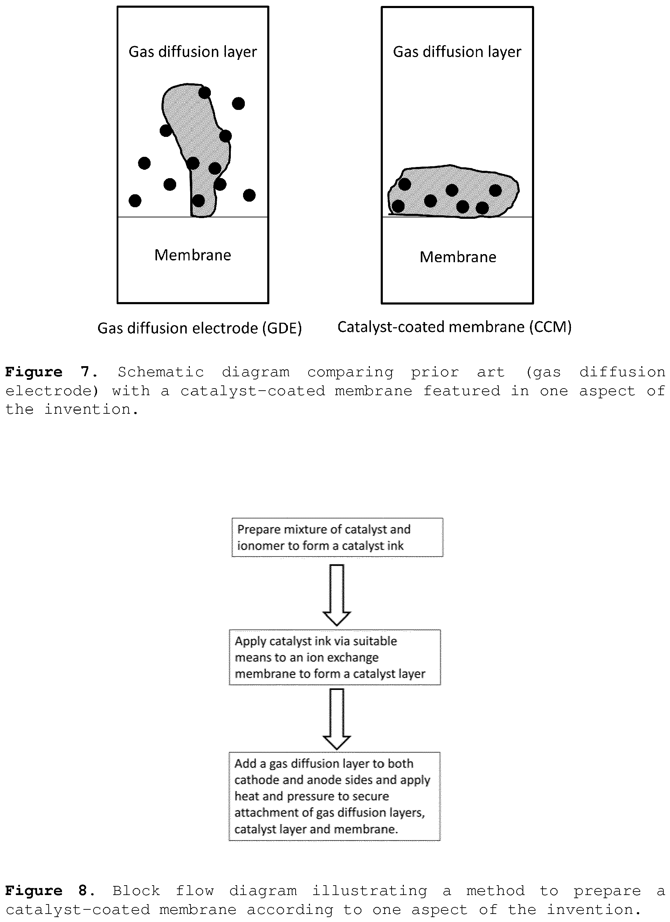

In yet another aspect of the invention, the catalyst is applied as a catalyst coated membrane. That way, catalyst material is concentrated in the vicinity of the membrane and has good ionic contact. Importantly, using this method the catalyst layer is thin, which is favorable because it minimizes decomposition of chemical compounds produced at the catalyst during the extraction process. Using a catalyst coated membrane for the production of chemical compounds, especially if such compounds are in the liquid form, is beneficial to decrease residence time of the compounds in the catalyst layer, and it also allows for improved catalyst utilization and reduced loadings. A schematic representation of a catalyst coated membrane and a gas diffusion electrode is shown in Figure 7. The catalyst coated membrane contains a suitable catalyst for the reaction. For example, if the reaction is oxygen reduction to hydrogen peroxide said catalyst could be silver, carbon, gold, Pt-Au, Pd-Au, Pt-Hg, Pd-Hg, Ag-Hg, Cu-Hg, Co-containing materials, other carbon-based structures, graphene or graphene-based compounds doped with transition metals (including but not limited to iron, nickel, cobalt, manganese, copper, silver, gold, platinum, palladium, iridium, ruthenium) or post-transition metals, porphyrins containing transition metals (including but not limited to iron, nickel, cobalt, manganese, copper, silver, gold, platinum, palladium, iridium, ruthenium) or post-transition metals or any combinations thereof. A catalyst coated membrane could also be combined with a gas diffusion electrode. The gas diffusion electrode can be catalyst-coated on either or both sides. In that manner, the catalyst layer is facing the membrane, the liquid solution or both.

Figure 8 shows a method to prepare a catalyst coated membrane. This method consists of three steps: first, preparing a mixture of catalyst ionomer to form a catalyst ink; second, applying the catalyst ink to the ion exchange membrane to form a catalyst layer; third adding gas diffusion layers to both sides of the membrane and applying a heat and pressure treatment to facilitate bonding of the gas diffusion layers to the membrane. Preferably, the temperature applied during the third stage is between 60 and 200 °C, and the pressure between 20 and 2000 kg/cm².

### Brief description of the drawings

Figure 1 represents a schematic diagram of an electrochemical cell as a membrane electrode assembly.
Figure 2 shows a schematic diagram of the electrochemical cell design with liquid between the gas disperser and the gas diffusion layer. The gas disperser provides reactants to the catalyst layer by convection of the gas.
Figure 3 shows experimental results for a membrane electrode assembly producing hydrogen peroxide by oxygen reduction at the cathode. The current densities for the same membrane electrode assembly with dissolved oxygen, and using the present invention are shown.
Figure 4 is a schematic representation of a gas delivery system to the membrane electrode assembly. The frame indicated as point 10 is mounted against a membrane electrode assembly, which is then pressed from the other side with a similar frame, point 12, that may or may not include gas delivery system. The gas is delivered directly into the electrodes through gas dispersers indicated by point 11, and a pillar on the opposite side provides for counter-pressure (point 13).
Figure 5 is a schematic showing the concept of a gas delivery system enclosing a membrane electrode assembly. Point number 14 indicates a volume used for either a flowing or stagnant liquid, point 15 indicates the gas disperser through a channel which may have fluidizing media at the end and point 1 indicates the membrane electrode assembly immersed in the liquid.
Figure 6 shows a schematic representation of a gas diffusion layer with openings featured in the present invention.
Figure 7 shows a schematic diagram for two membrane electrode assemblies prepared on two different ways. A gas diffusion electrode (prior art) and a catalyst coated membrane, disclosed in the current invention.
Figure 8 is a block flow diagram explaining how to prepare a catalyst coated membrane.
Figure 9 is a diagram that shows how the gas can be connected to one or more electrochemical cells in series.
Figure 10 is a diagram that shows how the gas can be recirculated in one or more electrochemical cells. With this method the gas can be delivered in any stoichiometry while controlling the utilization.
Figure 11 is a diagram showing how locally generated hydrogen peroxide can be added to a water line or reservoir for local use. Point number 16 indicates the hydrogen peroxide generator, consisting of the electrochemical cell described herein. Point 17 indicates the water input to the electrochemical cell, point 18 the water line containing hydrogen peroxide, point 19 a reservoir for storage of the hydrogen peroxide solution and point 20 an ultra-violet light or ozone source which may or may not be present.

### Examples

### Example 1

### Electrochemical synthesis of hydrogen peroxide

Electrochemical synthesis of hydrogen peroxide is promising for distributed generation of the chemical closer to the point of use. This would overcome limitations of the current method for industrial hydrogen peroxide synthesis, the anthraquinone process, a large-scale process, which can only be efficiently conducted at large scale chemical plants. From the production plant hydrogen peroxide is concentrated to 30-70% solutions (labelled as a hazardous material) and transported to the point of use, where it typically needs to be diluted to manageable concentrations. These could be in the ppm to few-% range. The transportation and dilution process results in extensive logistics and complexity for end-users, who despite the attractive properties of hydrogen peroxide as an effective and environmentally friendly chemical turn to other alternatives such as chlorine or formaldehyde for their processes. A method to generate hydrogen peroxide on-site would simplify use of the chemical by eliminating the logistics burden, and facilitate application of hydrogen peroxide directly at the required low concentrations and at high purity. This would also improve safety of the processes as the concentration of hydrogen peroxide would not exceed dangerous levels. Hydrogen peroxide synthesized this way could also be used to enhance dissolved oxygen levels in water or other liquids, while accurately controlling concentration.

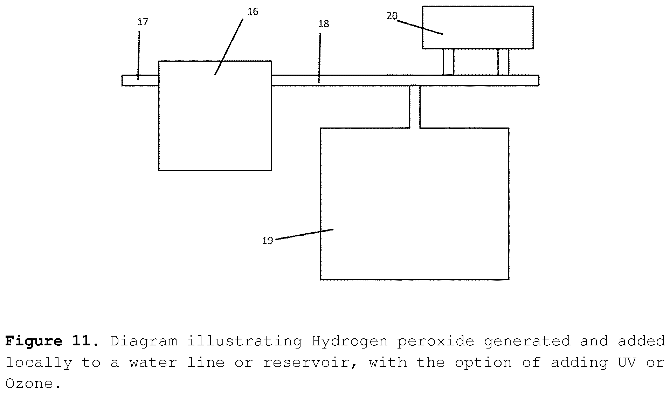

Some applications where hydrogen peroxide is used, and where its distributed generation can provide strong benefits, include bleaching in the pulp and paper industry, as a biocide or disinfectant in the agricultural and food industries (in aquaculture, animal drinking water, irrigation water treatment), in municipal and residential water treatment, in waste water treatment, in soil remediation, in swimming pools, or in healthcare for personal hygiene and as a surface and room disinfectant. Hydrogen peroxide produced locally can be introduced directly into a water line or stored in a reservoir prior to introduction into a water line, as shown in Figure 11. Further, in some of these applications, such as water treatment, hydrogen peroxide synthesized on site can also be combined with other reagents, such as ozone, or with ultra-violet radiation, which facilitate the formation of hydroxyl radicals with even stronger oxidizing potential. This prospect is illustrated in Figure 11. This would be very useful for disinfection applications, since radicals are known to be more powerful disinfection agents than hydrogen peroxide itself. Such an on-site hydrogen peroxide generator, possibly combined with other reagents such as ozone or ultra-violet light, can be used in swimming pools, room disinfection, laundry facilities and water treatment facilities. In some of these applications a sensoring option can be included to facilitate hydrogen peroxide generation in accordance with necessary concentrations levels further downstream. With suitable sensoring options, for instance by Oxidation Reduction Potential or hydrogen peroxide sensors, a feedback mechanism could be implemented to control the throughput of the hydrogen peroxide generators.

Hydrogen peroxide can be synthesized on-site through an electrochemical process using water and oxygen as reactants. Electrochemical generation can be achieved in compact devices generating on-site, on-demand hydrogen peroxide directly at the diluted concentrations required. Such a device has three distinct parts: anode, membrane and cathode. Water oxidation takes place at the anode, resulting in oxygen and protons. The protons are transported through an ion exchange membrane to the cathode, where they recombine with oxygen to yield hydrogen peroxide at the surface of an appropriate catalyst. The ion exchange membrane is typically a polymer electrolyte membrane, but could also be a hydrocarbon membrane or other suitable cation exchange membranes.

The half-cell reactions are as follows:
Anode:

   **2*H*₂*O* → *O*₂ + 4*H*⁺ + 4*e*⁻**
Cathode:

   **2*O*₂ + 4*H*⁺ + 4*e*⁻ → 2*H*₂*O*₂**

It is important to minimize hydrogen peroxide decomposition, which can take place chemically or electrochemically:

**2*H*₂*O*₂ → 2*H*₂*O* + *O*₂**

***H₂O₂* + 2*H*⁺ + 2*e*⁻ → 2*H*₂*O***

Other proton sources than water can be used at the anode, for example hydrogen, methane, methanol etc. These are evident to those skilled in the art.

Using the present invention in an electrochemical cell for hydrogen peroxide synthesis shows improved properties. The presence of a liquid near and through the cathode gas diffusion layer decreases residence time of hydrogen peroxide in the vicinity of the electrode, which minimizes its decomposition. Simultaneously, an oxygen containing gas is delivered to the cathode electrode, as depicted in Figure 2 and Figure 4. This induces fast movement of both liquid and gas species in the vicinity of the catalyst layer, which favors fast mass transport conditions. These are key to two goals: a) effective transport of oxygen to the electrode; b) decrease the residence time of the produced hydrogen peroxide at the electrode, minimizing its decomposition either by chemical or electrochemical means.

In addition, if liquid is flowing near and/or through the gas diffusion layer it further favors fast mass transport, and it is preferred over non-flowing liquid. Preferably the liquid is composed of more than 90 % of water, and even more preferably it is water.

In another aspect of the invention, air (or another suitable oxygen source) is fed to the cathode side of the cells in the close vicinity, in a manner that gas is directed into the gas diffusion layer and to the catalyst layer, depicted in Figure 2 and Figure 5. This enhances mass transport of oxygen species to the cathode side of the cell and facilitates high current densities.

In yet another aspect, the gas diffusion layer used at the cathode side is modified to facilitate extraction of the produced hydrogen peroxide, shown in Figure 6. These holes facilitate the transport of hydrogen peroxide out of the catalyst and gas diffusion layers because the dense carbon fiber network forming the gas diffusion layer has been removed. In addition, the holes are easily filled up with liquid which is also beneficial for removal of hydrogen peroxide. A gas diffusion layer patterned in this manner could be coated with a suitable catalyst.

In another aspect, the cathode electrode consists of a catalyst coated membrane, as seen in Figure 7. This ensures the catalyst layer is in immediate contact with the membrane, and it avoids dispersion of the catalyst ink into the gas diffusion layer. This is preferred because the catalyst itself may decompose hydrogen peroxide after it has been extracted. Limiting the catalyst layer at the region neighboring the membrane minimizes this effect. Suitable catalyst materials include those known for oxygen reduction to hydrogen peroxide, such as silver, carbon, gold, Pt-Au, Pd-Au, Pt-Hg, Pd-Hg, Ag-Hg, Cu-Hg, Co-containing materials, graphene or other carbon-based structures, graphene doped with transition or post-transition metals, porphyrins containing transition metals or post-transition metals or any combinations thereof.

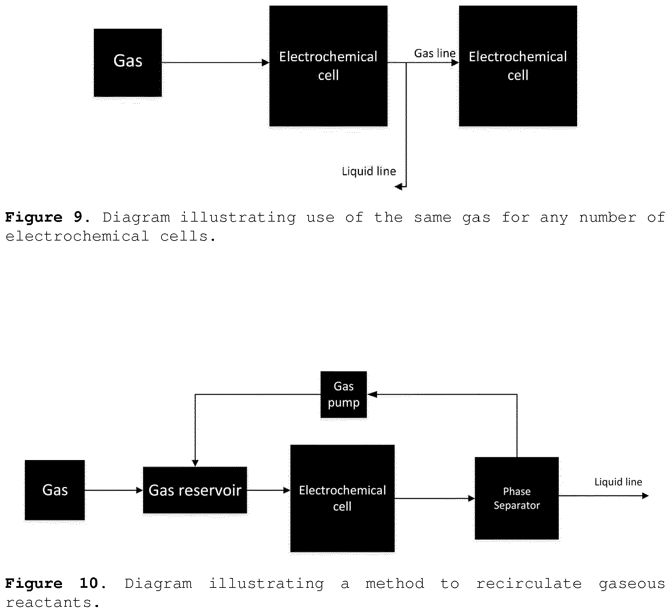

Further described is how the oxygen-containing gas is delivered to the cathode and exits the housing through an outlet. At this point the gas may be directed back into the cathode of the same cell, or to the cathode of another cell, as described in Figure 9. This is particularly beneficial when using a gas with a higher concentration of oxygen than air (for example gas obtained from an oxygen concentrator). This enables utilization of any oxygen which was not used in the initial electrochemical cell. If there is only one cell, gas can be accumulated in a gas tank, fed into the cell and once it exits the cell recirculated into the gas tank from which it can be reintroduced into the cell, as shown in Figure 10. This same method can also be utilized when there are several electrochemical cells, in a manner that gas is fed into the first cell, exits the first cell, and it enters the second cell, from where it is directed into the third cell or recirculated into the gas tank. A phase separator to separate water and gas may be utilized between the two cells. This method can be utilized for an indefinite number of cells, the only limiting factor being the pressure of the gas.

The oxygen gas produced at the anode of the electrochemical cells can also be merged with the cathode gas feed at any point of the gas lines. This may enable even higher oxygen concentrations at the cathode gas feeds.

In an example cell, the cathode is made of an ink consisting of a suitable catalyst, ionomer solution, a suitable solvent (typically an alcohol) and water. The solid content of the ink has a catalyst ratio of between 1:5 to 4:5, an ionomer ratio between 1:5 to 4:5 and may contain PTFE in ratios between 0 to 4:5, preferably between 1:20 to 4:5. The ink may also contain a suitable surfactant, such as Triton, a quaternary ammonium compound or other suitable polymers in ratios between 0 to 4:5. The ink is applied onto a gas diffusion layer (with the resulting electrode known as gas diffusion electrode). Further, the coating can take place on one side or on both sides of the gas diffusion layer. Alternatively, the cathode can also consist of a catalyst coated membrane, where the ink is sprayed directly on an ion exchange membrane and a gas diffusion layer is added afterwards.

The anode in this example is a water oxidation anode, and is prepared in the catalyst coated membrane configuration well-known to those versed in the art (see for example Handbook of Fuel Cells: Fundamentals, Technology and Applications, Wiley VCH, 2014). Other anode reactions than water oxidation, including suitable materials, could be used without affecting the present example.

Anode and cathode are pressed on either side of an ion exchange membrane and incorporated into the electrochemical cell housing. The compartments of the housing are filled with water, and an oxygen-containing gas is injected into the cathode side of the system. In this example the distance between the gas disperser and the cathode gas diffusion layer is <1 mm. The experiment takes place at room temperature, and the pressure of the gas can vary between 0 and 50 bar, preferably between 0 and 5 bar. In a typical experiment a potential difference between 0.5 and 2.5 V, preferably between 1.5 and 2.2 V, is applied between anode and cathode, and current is observed indicating electrochemical reactions are taking place. Preferably the current is above 30 mA/cm², and even more preferably above 50 mA/cm². The experiment is let run for a certain amount of time, while water is flowing through both anode and cathode compartments, and the hydrogen peroxide concentration produced at the cathode is determined via a suitable method, which could be permanganate titration or indicator strips. This hydrogen peroxide can be accumulated in a reservoir for its later use, or utilized directly.

## Claims

1. Apparatus or device for producing a chemical compound by electrochemical means, comprised of one or more electrochemical cells in the membrane electrode assembly (1) configuration comprising
- at least one ion exchange membrane (4),
- at least one cathode with suitable catalysts (5, 6),
- at least one anode with suitable catalysts (2, 3), whereas the cathode (5, 6) and the anode (2,3) are applied as catalyst-coated membranes or gas diffusion electrodes or a combination of the two pressed or coated against either sides of the ion exchange membrane (4),
- at least one gas diffusion layer or current collector on the cathode side (6) and
- at least one gas diffusion layer or current collector on the anode side (2),
wherein the cathode or anode or both sides of the cell are at least partly immersed in a liquid at least on the side of the membrane requiring a gas reactant, and at least one gas reactant which during operation of the apparatus is fed to the cathode or anode or both, wherein a gas disperser (8, 15) comprising a fluidizing media to enhance dispersion of the gas is placed in contact with the cathode or anode or both sides of the cell; and wherein the liquid is water.

2. Apparatus with a gas disperser (8, 15) according to claim 1 wherein during operation of the apparatus the gas is directed into the gas diffusion layer (2, 6) and through to a catalyst layer (3, 5).

3. Apparatus or device according to claims 1-3 wherein an oxygen source is air or an oxygen supply.

4. Apparatus or device according to claims 1-3 wherein the cathode is a gas diffusion electrode.

5. Apparatus with a gas diffusion electrode according to claim 4 wherein the cathode catalyst is chosen from the group including silver, carbon, gold, Pt-Au, Pd-Au, Pt-Hg, Pd-Hg, Ag-Hg, Cu-Hg, Co-containing materials, graphene or other carbon-based structures, graphene doped with transition metals, porphyrins containing transition metals.

6. Apparatus or device according to claims 1-3 wherein the anode catalyst is chosen from the group including iridium, ruthenium, platinum, gold or mixtures thereof and is applied as a catalyst-coated membrane.

7. Apparatus or device according to claims 1-3 wherein the cathode is applied as a catalyst coated membrane.

8. Apparatus with a catalyst-coated membrane according to claim 7 wherein the cathode catalyst is chosen from the group including silver, carbon, gold, Pt-Au, Pd-Au, Pt-Hg, Pd-Hg, Ag-Hg, Cu-Hg, Co-containing materials, graphene or other carbon-based structures, graphene doped with transition metals, porphyrins containing transition metals.

9. Method for producing a chemical compound by electrochemical means using one or more electrochemical cells in the membrane-electrode assembly (1) configuration comprising at least one ion exchange membrane (4), at least one cathode with suitable catalysts (5, 6), at least one anode with suitable catalysts (2,3), whereas the cathode and the anode are catalyst-coated membranes or gas diffusion electrodes or a combination of the two pressed or coated against either sides of the ion exchange membrane (4), at least one gas diffusion layer or current collector on the cathode side (6) and at least one gas diffusion layer or current collector on the anode side (2), wherein the cathode or anode or both sides of the cell are at least partly immersed in a liquid, at least on the side of the membrane requiring a gas reactant, **characterized in that** a gas disperser (8, 15) comprising a fluidizing media to enhance dispersion of the gas is placed in contact with the cathode or anode or both sides of the cell and feeds a reactant gas through the liquid directly to the cathode or anode or both sides of the cell, wherein the liquid is water.

10. Method according to claim 9 wherein the chemical compound is hydrogen peroxide produced by oxygen reduction at the cathode side of the cell.

## Patentansprüche

1. Apparat oder Vorrichtung zur Herstellung einer chemischen Verbindung auf elektrochemischem Wege, bestehend aus einer oder mehreren elektrochemischen Zellen in der Konfiguration der Membran-ElektrodenAnordnung (1), umfassend
- mindestens eine lonenaustauschmembran (4),
- mindestens eine Kathode mit geeigneten Katalysatoren (5, 6),
- mindestens eine Anode mit geeigneten Katalysatoren (2, 3), wobei die Kathode (5, 6) und die Anode (2, 3) als katalysatorbeschichtete Membranen oder Gasdiffusionselektroden oder eine Kombination aus beiden ausgeführt sind und gegen beide Seiten der lonenaustauschmembran (4) gepresst oder beschichtet sind,
- mindestens eine Gasdiffusionsschicht oder Stromkollektor auf der Kathodenseite (6) und
- mindestens eine Gasdiffusionsschicht oder Stromkollektor auf der Anodenseite (2),
wobei die Kathode oder Anode oder beide Seiten der Zelle mindestens teilweise in eine Flüssigkeit mindestens auf der Seite der Membran eingetaucht sind, die einen Gasreaktanten erfordert, und
mindestens einen Gasreaktanten, der während des Betriebs der Vorrichtung der Kathode oder Anode oder beiden zugeführt wird, wobei ein Gasdispergierer (8, 15), der ein Fluidisierungsmedium zur Verbesserung der Dispersion des Gases umfasst, in Kontakt mit der Kathode oder Anode oder beiden Seiten der Zelle angeordnet ist; und wobei die Flüssigkeit Wasser ist.

2. Vorrichtung mit einem Gasdispergierer (8, 15) nach Anspruch 1, wobei während des Betriebs der Vorrichtung das Gas in die Gasdiffusionsschicht (2, 6) und durch diese hindurch zu einer Katalysatorschicht (3, 5) geleitet wird.

3. Apparat oder Vorrichtung nach Ansprüchen 1-3, wobei eine Sauerstoffquelle Luft oder eine Sauerstoffversorgung ist.

4. Apparat oder Vorrichtung nach Ansprüchen 1-3, wobei die Kathode eine Gasdiffusionselektrode ist.

5. Vorrichtung mit einer Gasdiffusionselektrode nach Anspruch 4, wobei der Kathodenkatalysator aus der Gruppe ausgewählt ist, die Silber, Kohlenstoff, Gold, Pt-Au, Pd-Au, Pt-Hg, Pd-Hg, Ag-Hg, Cu-Hg, Co enthaltende Materialien, Graphen oder andere Strukturen auf Kohlenstoffbasis, mit Übergangsmetallen dotiertes Graphen und Übergangsmetalle enthaltende Porphyrine umfasst.

6. Apparat oder Vorrichtung nach Ansprüchen 1-3, wobei der Anodenkatalysator aus der Gruppe ausgewählt ist, die Iridium, Ruthenium, Platin, Gold oder Mischungen davon einschließt, und als eine katalysatorbeschichtete Membran aufgebracht ist.

7. Apparat oder Vorrichtung nach Ansprüchen 1-3, bei dem die Kathode als katalysatorbeschichtete Membran aufgebracht ist.

8. Vorrichtung mit einer katalysatorbeschichteten Membran nach Anspruch 7, wobei der Kathodenkatalysator aus der Gruppe ausgewählt ist, die Silber, Kohlenstoff, Gold, Pt-Au, Pd-Au, Pt-Hg, Pd-Hg, Ag-Hg, Cu-Hg, Co-enthaltende Materialien, Graphen oder andere Strukturen auf Kohlenstoffbasis, mit Übergangsmetallen dotiertes Graphen , übergangsmetallhaltige Porphyrine einschließt.

9. Verfahren zur Herstellung einer chemischen Verbindung auf elektrochemischem Wege unter Verwendung einer oder mehrerer elektrochemischer Zellen in der Konfiguration einer Membran-ElektrodenAnordnung (1), die mindestens eine lonenaustauschmembran (4), mindestens eine Kathode mit geeigneten Katalysatoren (5, 6), mindestens eine Anode mit geeigneten Katalysatoren (2, 3) umfasst, während die Kathode und die Anode katalysatorbeschichtete Membranen oder Gasdiffusionselektroden oder eine Kombination der beiden sind, die gegen beide Seiten der lonenaustauschmembran (4) gepresst oder beschichtet sind, mindestens eine Gasdiffusionsschicht oder Stromkollektor auf der Kathodenseite (6) und mindestens eine Gasdiffusionsschicht oder Stromkollektor auf der Anodenseite (2), wobei die Kathode oder Anode oder beide Seiten der Zelle zumindest teilweise in eine Flüssigkeit eingetaucht sind, zumindest auf der Seite der Membran, die einen Gasreaktanten benötigt, **dadurch gekennzeichnet, dass** ein Gasdispergierer (8, 15), der ein Fluidisierungsmedium zur Verbesserung der Dispersion des Gases umfasst, mit der Kathode oder Anode oder beiden Seiten der Zelle in Kontakt gebracht wird und ein Reaktionsgas durch die Flüssigkeit direkt zur Kathode oder Anode oder beiden Seiten der Zelle (1) zuführt, wobei die Flüssigkeit Wasser ist.

10. Verfahren nach Anspruch 9, wobei die chemische Verbindung Wasserstoffperoxid ist, das durch Sauerstoffreduktion an der Kathodenseite der Zelle erzeugt wird.

## Revendications

1. Appareil ou dispositif pour produire un composé chimique par des moyens électrochimiques composés d'une ou de plusieurs cellules électrochimiques dans la configuration de l'ensemble d'électrode à membrane (1) comprenant :
- au moins une membrane échangeuse d'ions (4),
- au moins une cathode avec des catalyseurs appropriés (5, 6),
- au moins une anode avec des catalyseurs appropriés (2, 3), tandis que la cathode (5, 6) et l'anode (2, 3) sont appliquées comme des membranes enduites de catalyseur ou des électrodes de diffusion de gaz ou une combinaison des deux pressés ou revêtues contre l'un ou l'autre côté de la membrane échangeuse d'ions (4),
- au moins une couche de diffusion de gaz ou un collecteur de courant sur le côté cathode (6) et
- au moins une couche de diffusion de gaz ou un collecteur de courant sur le côté anode (2),
cependant que la cathode ou l'anode ou les deux côtés de la cellule sont au moins partiellement immergés dans un liquide au moins sur le côté de la membrane nécessitant un réactif gazeux qui est conduit, pendant le fonctionnement de l'appareil, à la cathode ou à l'anode ou aux deux, cependant qu'un disperseur de gaz (8, 15) qui comprend un milieu de fluidisation pour augmenter la dispersion du gaz est placé en contact avec la cathode ou l'anode ou les deux côtés de la cellule et cependant que le liquide est de l'eau.

2. Appareil avec un disperseur de gaz (8, 15) selon la revendication 1, cependant que, pendant le fonctionnement de l'appareil, le gaz est dirigé dans la couche de diffusion de gaz (2, 6) et à travers une couche de catalyseur (3, 5).

3. Appareil ou dispositif selon les revendications 1-2, une source d'oxygène étant une alimentation en air ou en oxygène.

4. Appareil ou dispositif selon les revendications 1 à 3, la cathode étant une électrode de diffusion de gaz.

5. Appareil avec une électrode de diffusion de gaz selon la revendication 4, cependant que le catalyseur de la cathode est sélectionné dans le groupe comprenant l'argent, le carbone, l'or, Pt-Au, Pd-Au, Pt-Hg, Pd-Hg, Ag-Hg, Cu-Hg, des matériaux contenant du Co, du graphène ou d'autres structures à base de carbone, du graphène dopé avec des métaux de transition, des métaux de transition contenant des porphyrines.

6. Appareil ou dispositif selon les revendications 1 à 3, cependant que le catalyseur de l'anode est sélectionné dans le groupe comprenant l'iridium, le ruthénium, le platine, l'or ou des mélanges de ceux-ci et est appliqué en tant que membrane enduite de catalyseur.

7. Appareil ou dispositif selon les revendications 1 à 3, cependant que la cathode est appliquée en tant que membrane enduite de catalyseur.

8. Appareil avec une membrane enduite de catalyseur selon la revendication 7, cependant que le catalyseur de la cathode est sélectionné dans le groupe comprenant l'argent, le carbone, l'or, Pt-Au, Pd-Au, Pt-Hg, Pd-Hg, Ag-Hg, Cu-Hg, des matériaux contenant du Co, du graphène ou d'autres structures à base de carbone, du graphène dopé avec des métaux de transition, des métaux de transition contenant des porphyrines.

9. Procédé pour produire un composé chimique par des moyens électrochimiques qui utilisent une ou plusieurs cellules électrochimiques dans la configuration de l'ensemble d'électrode à membrane (1) comprenant au moins une membrane échangeuse d'ions (4), au moins une cathode avec des catalyseurs appropriés (5, 6), au moins une anode avec des catalyseurs appropriés (2, 3), tandis que la cathode et l'anode sont des membranes enduites de catalyseur ou des électrodes de diffusion de gaz ou une combinaison des deux pressées ou revêtues contre l'un ou l'autre côté de la membrane échangeuse d'ions (4), au moins une couche de diffusion de gaz ou un collecteur de courant sur le côté cathode (6) et au moins une couche de diffusion de gaz ou un collecteur de courant sur le côté anode (2), cependant que la cathode ou l'anode ou les deux côtés de la cellule sont au moins partiellement immergés dans un liquide au moins sur le côté de la membrane nécessitant un réactif gazeux, **caractérisé en ce qu'**un disperseur de gaz (8, 15) qui comprend un milieu de fluidisation pour augmenter la dispersion du gaz est placé en contact avec la cathode ou l'anode ou les deux côtés de la cellule et conduit un gaz réactif à travers le liquide directement vers la cathode ou l'anode ou les deux côtés de la cellule, cependant que le liquide est de l'eau.

10. Procédé selon la revendication 9, le composé chimique étant du peroxyde d'oxygène produit par réduction d'oxygène sur le côté cathode de la cellule.
